(19) Europäisches Patentamt / European Patent Office / Office européen des brevets

(11) **EP 3 315 106 B1**

(12) **EUROPEAN PATENT SPECIFICATION**

(45) Date of publication and mention
of the grant of the patent:
**07.08.2019 Bulletin 2019/32**

(51) Int Cl.:
***A61F 13/532*** *(2006.01)*       ***A61F 13/537*** *(2006.01)*
***A61F 13/551*** *(2006.01)*

(21) Application number: **16196510.8**

(22) Date of filing: **31.10.2016**

(54) **ABSORBENT ARTICLE WITH AN INTERMEDIATE LAYER COMPRISING CHANNELS AND BACK POCKET**

ABSORBIERENDER ARTIKEL MIT ZWISCHENSCHICHT MIT KANÄLEN UND RÜCKENTASCHE

ARTICLE ABSORBANT COMPORTANT UNE COUCHE INTERMÉDIAIRE COMPRENANT DES CANAUX ET UNE POCHE ARRIÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.05.2018 Bulletin 2018/18**

(73) Proprietor: **The Procter & Gamble Company Cincinnati, OH 45202 (US)**

(72) Inventors:
 • **Garcia, Julien René**
   **65824 Schwalbach am Taunus (DE)**
 • **Bianchi, Ernesto Gabriel**
   **65824 Schwalbach am Taunus (DE)**
 • **Endres, Joerg**
   **65824 Schwalbach am Taunus (DE)**
 • **Martynus, Cornelia Beate**
   **65824 Schwalbach am Taunus (DE)**
 • **Stelzig, Lutz**
   **65824 Schwalbach am Taunus (DE)**

(74) Representative: **L'Huillier, Florent Charles
Procter & Gamble Service GmbH
IP Department
Sulzbacher Straße 40-50
65824 Schwalbach am Taunus (DE)**

(56) References cited:
**WO-A1-2015/031225      WO-A1-2015/031229
WO-A1-2015/183282**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The invention relates to personal hygiene absorbent articles such as, but not limited to, baby diapers, training pants, feminine pads and adult incontinence products. The absorbent articles of the invention comprise in a first aspect a fibrous intermediate layer that comprises at least two channels connected to a back pocket.

BACKGROUND OF THE INVENTION

Absorbent articles for personal hygiene are designed to absorb and contain body exudates, in particular large quantity of urine. These absorbent articles typically comprise several layers that provide different functions, in particular a topsheet for quickly acquiring the fluid and that should feel soft on the wearer's skin, a backsheet for protection the wearer's clothes, and an absorbent core for retaining fluid. It is also known to provide one or more intermediate layers between the topsheet and the absorbent core. These intermediate layers are also called "wicking layer", "surge layer", "acquisition layer", "liquid management system" (LMS) or "distribution layer" and will be referred to herein as intermediate layers. These intermediate layers are designed to quickly acquire and distribute the fluid away from the topsheet and direct the fluid into the absorbent core for storage. Intermediate layers typically do not comprise superabsorbent material, unlike the absorbent core.

WO94/23761 (Payne et al.) for example discloses an upper acquisition layer comprising an homogeneous composition of hydrophilic fibrous material comprising stiffened, twisted, and curled cellulose fibers and having a densified distribution zone. Other examples referred to as surge layers are found for example in US5,486,166 and US5,490,846 (Bishop et al.). Articles having two intermediate layers or more, in particular an acquisition layer having a high capillarity which pulls the fluid quickly away from the topsheet and a distribution layer having a larger void area to distribute the fluid over a large surface over the core, are also known. WO2014/93323 (Bianchi et al.) for example discloses an absorbent article with a profiled acquisition-distribution system. Other exemplary references disclosing such intermediate layers are US2008/0312621 and US2008/0312622 (both Hundorf et al.), WO99/17679 (Everett et al.). Absorbent articles comprising channels in the absorbent core and partially overlapping channels in a liquid management system have been more recently disclosed in WO2015/31225, WO2015/31229, WO2015/31243, WO2015/31256 (Roe et al.).

WO2015/031225 (Roe et al.) discloses an absorbent article comprising a liquid management system (LMS) and an absorbent core disposed intermediate a topsheet and a backsheet. The liquid management system disclosed may comprise two longitudinally extending channels as well as a pocket disposed towards the rear of the article. The channels and the pocket extend substantially through the liquid management system, but are not connected to each other. The absorbent core may also comprise channels that may or may not at least partially overlap with the channels of the liquid management system.

While the known absorbent articles can have good overall properties, there is a continuous need to improve containment performance while keeping the cost of manufacture as low as possible. The present invention addresses these problems.

SUMMARY OF THE INVENTION

The present invention is directed to an absorbent article for personal hygiene having a longitudinal axis extending from a front edge to a back edge of the article. The absorbent article comprises a fluid permeable topsheet on a wearer-facing side, a fluid-impermeable backsheet on a garment-facing side, an absorbent core comprising an absorbent material between the topsheet and backsheet, and an intermediate layer between the topsheet and the absorbent core. The intermediate layer comprises a fibrous material. The intermediate layer comprises a first and second longitudinally-extending channels disposed respectively on opposite side of the longitudinal axis. The fibrous layer further comprises a back pocket. The channels and the back pocket are substantially free for absorbent material. The back pocket is connected to the first and second channels to form an unitary area substantially free of fibrous material. This and other aspects of the invention will be described in the following description.

BRIEF DESCRIPTION OF THE DRAWINGS

Fig. 1 is a top view of an exemplary article of the invention in the form of a taped diaper which has been pulled flat in open configuration, with the intermediate layer shown in dotted line;
Fig. 2 shows an exploded view of the main components of the taped diaper of Fig. 1;
Fig. 3 shows a transversal cross-section of the diaper of Fig. 1 taken in the area where the channels are present;
Fig. 4 shows a transversal cross-section of the diaper of Fig. 1 taken in the area where the back pocket is present;
Fig. 5 shows a top view of the intermediate layer of the diaper of Fig. 1 in isolation;
Fig. 6 shows a top view of an alternative intermediate layer according to the invention.

## DETAILED DESCRIPTION OF THE INVENTION

### General description of an absorbent article

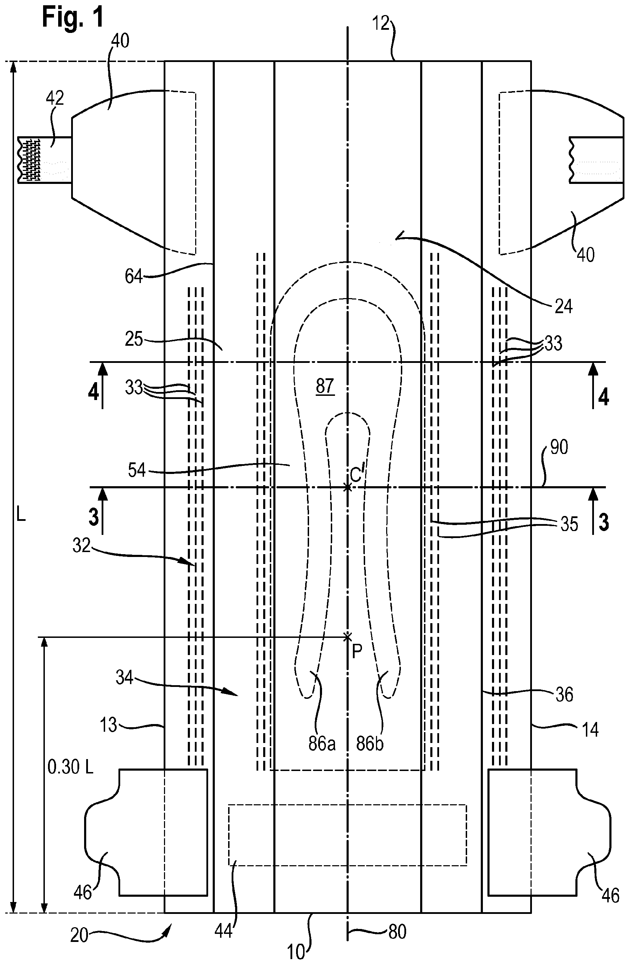

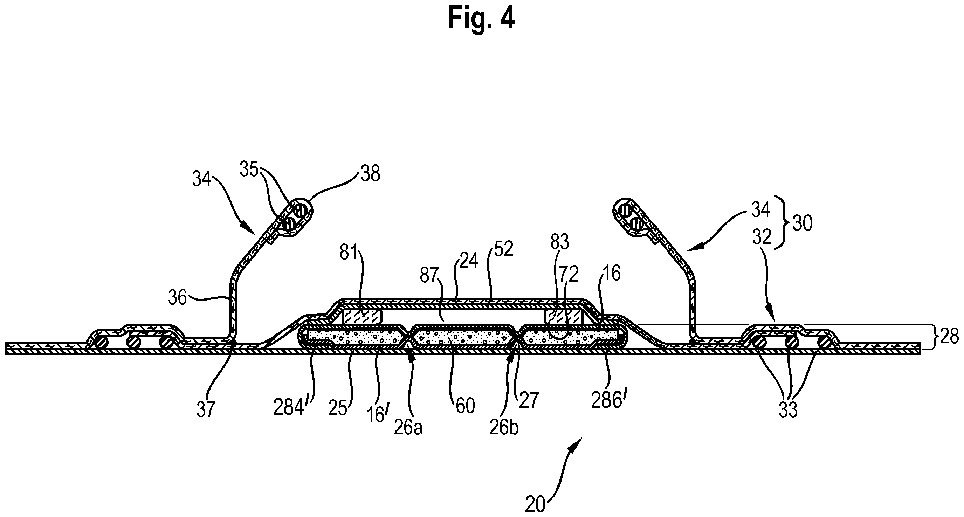

An exemplary absorbent article according to the invention in the form of a baby taped diaper 20 is represented in Figs. 1-4. This diaper 20 is shown for illustration purpose only, as the invention may be used for making a wide variety of diapers or other absorbent articles such as pant diapers, training pants, adult incontinence pants or feminine sanitary pads. The present invention may be especially useful for diapers for new born babies aged less than 1 year, in particular less than 6 months, as these babies may have especially runny or pasty bowel movements that can be efficiently acquired in the back pocket of the intermediate layer. The invention can however also be useful for more pasty and solid bowel movement that can be stored in the void volume of the back pocket.

The absorbent article 20 comprises a front edge 10, a back edge 12, and two longitudinally-extending side (lateral) edges 13, 14. The front edge 10 is the edge of the article which is intended to be placed towards the front of the wearer when worn, and the back edge 12 is the opposite edge. The absorbent article is notionally divided by a longitudinal axis 80 extending along a longitudinal direction from the middle of the front edge to the middle of the back edge of the article and dividing the article in two substantially symmetrical halves relative to this axis, when viewing the article from the wearer-facing side in a flat out configuration, as exemplarily shown in Fig. 1. If some parts of the article are under tension due to elasticized components, the article may be typically flattened using clamps along the periphery of the article and/or a sticky surface, so that the article can be pulled taut so as to be substantially flat. Closed articles such as diaper pants or training pants or adult incontinent pants may be cut open along the side seams to apply them on a flat surface. Unless otherwise indicated, dimensions and areas disclosed herein apply to the article in this flat-out configuration. The article has a length L as measured along the longitudinal axis 80 from the back edge to the front edge.

The absorbent article can also be notionally divided by a transversal axis 90 into a front region and a back region of equal length measured on the longitudinal axis, when the article is in such a flat state as shown on Fig. 1. The transversal axis 90 is perpendicular to the longitudinal axis 80 and placed at half the length of the article. The intersection of the longitudinal axis 80 and the transversal axis 90 is defined herein as the centerpoint C of the article. The P point is further defined herein as the point on the longitudinal axis situated at a distance of 0.30 of the length of the article (L) from the front edge of the article. The P point typically corresponds in many articles to a zone of relatively high volume of fluid insult.

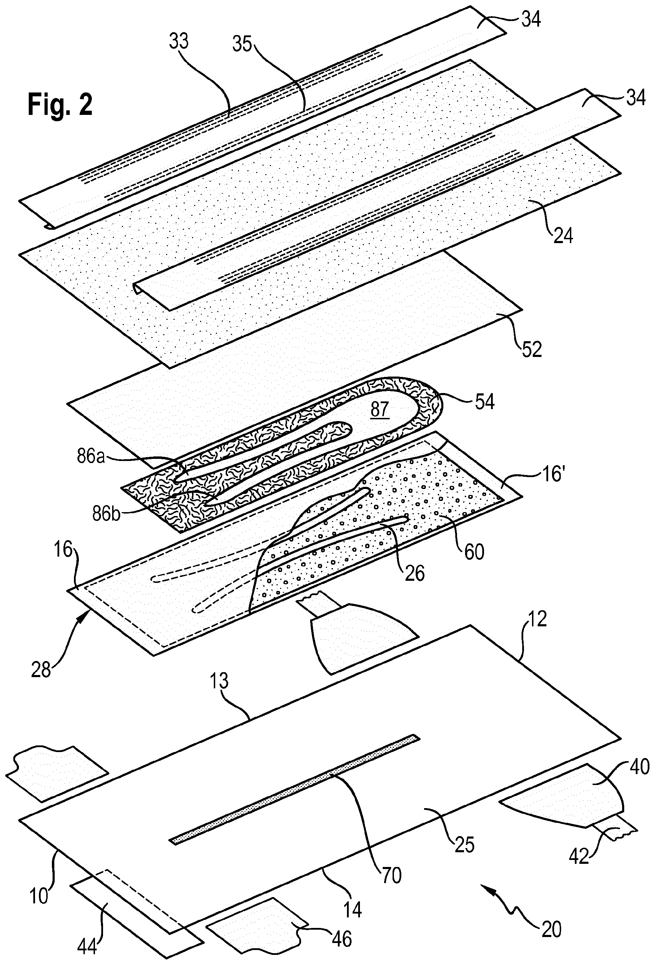

The absorbent article 20 comprises a liquid permeable topsheet 24, a liquid impermeable backsheet 25 and an absorbent core 28 between the topsheet and the backsheet. The intermediate layer 54 of the invention is disposed between the absorbent core and the topsheet, as better seen on Fig. 2. The absorbent article may, as exemplary represented, also comprise an acquisition layer 52 directly underneath the topsheet. The acquisition layer 52 is optional. The intermediate layer 54 may be between such acquisition layer and the absorbent core or may be disposed directly between the topsheet and the absorbent core if no acquisition layer is present. The intermediate layer 54 is a fibrous layer which may typically comprise more or less loosely bonded hydrophilic fibers that can quickly absorb and distribute fluid over a large area. The intermediate layer of the invention comprises a pair of longitudinally-extending channels 86a, 86b which are connected to a back pocket 87. These channels and the back pocket are substantially free of absorbent material. The intermediate layer 54 will be further discussed in details in the next section.

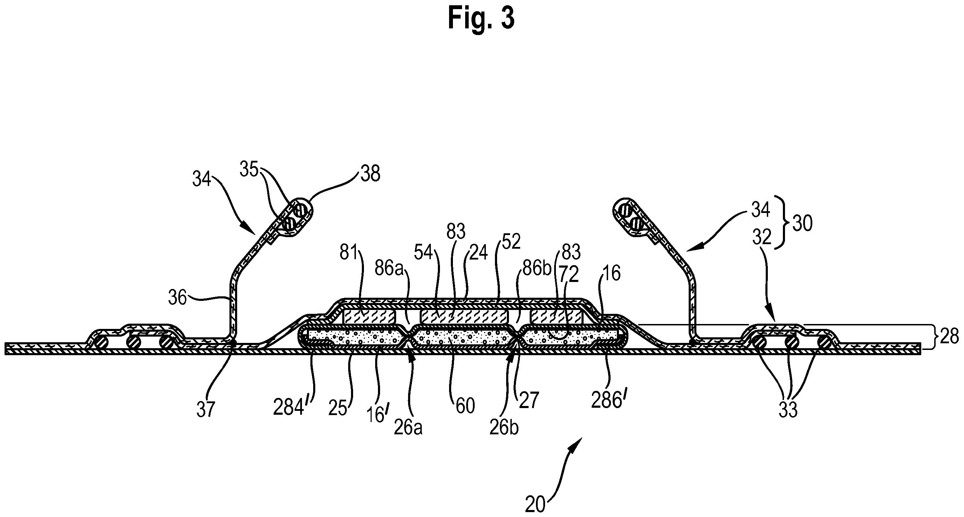

The invention is not limited to particular diaper components unless specified otherwise: any typical topsheet, backsheet or absorbent core may be used in the invention. The absorbent core may advantageously comprise a pair of channel areas 26a, 26b. The channels of the intermediate layer can advantageously cooperate with such channel areas present in an absorbent core, especially when these are at least partially superposed with the channels of the intermediate layer as illustrated in Fig. 3. The channel areas of the core may be substantially free of absorbent material and the top side of the core wrap may be optionally bonded to the bottom side of the core wrap through these channel areas to provide for permanent channels in dry and wet state. The absorbent core may however also comprise channels without core wrap bonds, or may be devoid of channel areas.

Other typical diaper components are represented on Figs. 1-2 such as elasticized gasketing cuffs 32, upstanding barrier leg cuffs 34, fastening tabs 42 and landing zone 44 and an urine indicator 70. These further components will be exemplified in more details further below. The absorbent article may also comprise other typical components, which are not represented in the Figures, such as a back elastic waist feature, a front elastic waist feature, transverse barrier cuffs, a lotion application, etc..

The topsheet 24, the backsheet 25, the absorbent core 28 and the other article components may be assembled in a variety of well-known configurations, in particular by gluing, fusion and/or pressure bonding. The absorbent article is advantageously thin. The article may for example have a caliper of from 1.0 mm to 8.0 mm, in particular from 1.5 mm to 6.0 mm, at the centerpoint C as measured using the Absorbent Article Caliper Test described below.

### Intermediate layer 54

The absorbent article comprises an intermediate layer 54 between the topsheet 24 and the absorbent core 28. One of the functions of the intermediate layer is to spread the insulting fluid liquid over a larger surface within the article so that the absorbent capacity of the core can be more efficiently used. Typically, intermediate layers are made of a fibrous material based on synthetic or cellulosic fibers and have a relatively low density. There may be other layers between the intermediate layer and any of the topsheet and the absorbent core, for example an acquisition layer 52 immediately under the topsheet. The fibrous material used to make the intermediate layer may in particular have a Water Retention Value of from 2 to 60, in particular from 3 to 40, more particularly from 4 to 20, as measured by the Water Retention Value Procedure described further below. The density of the intermediate layer where the fibrous material is present may vary depending on the compression of the article, but may typically range from 0.03 g/cm$^3$ to 0.25 g/cm$^3$, in particular from 0.05 g/cm$^3$ to 0.15 g/cm$^3$, measured at 0.30 psi (2.07kPa), and may be for example measured at the centerpoint C and/or the P point. The fibrous material may in particular comprise or consists of cross-linked cellulose fibers, as will be detailed further below, but other typical fibrous material can also be used.

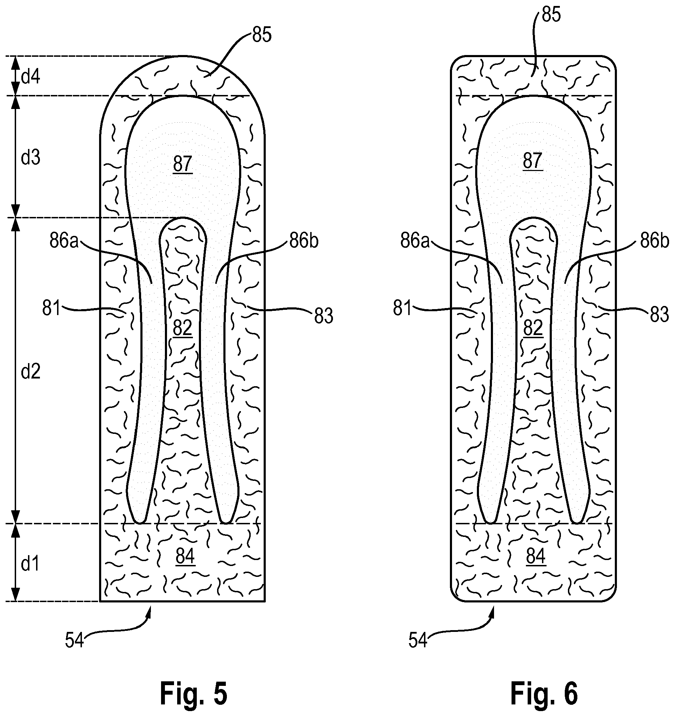

The exemplary intermediate layer of the diaper of Figs.1-4 is shown in isolation on Fig. 5. The smaller straight edge of the intermediate layer is the edge closest to the front edge of the article in the article, while the opposite rounded edge is oriented towards the back of the article. The intermediate layer may profiled, that it is the basis weight (amount of material per unit of surface) varies in the areas of the intermediate layer comprising the fibrous material. The layer may be longitudinally and/or transversally profiled, with typically higher basis weight towards the front and the middle of intermediate layer relative to the back and sides of the intermediate layer.

The intermediate layer 54 of the invention comprises a first longitudinally-extending channel 86a substantially free of fibrous material on one side of the longitudinal axis, and a second longitudinally-extending channel 86b on the other side of the longitudinal axis. The area between the channels comprising fibrous material is a central area 82 having a length d2. By "substantially free of fibrous material", it is meant that some fibers may be present in the channels or the back pocket, for example due to unavoidable contamination, but that these fibers do not provide a significant fluid distribution function nor prevent the channels and the back pocket from acquiring and distributing runny bowel movement along their length, as described below.

One function of the channels is to provide for the quick transfer of an incoming fluid into the underlying layer, typically the core. The channels may also provide more flexibility to the absorbent articles. The channels may typically extend to a significant length of the article. The length d2 of the channels may be for example at least 10% that of the length L of the absorbent article, in particular from 10% to 50% of the length L. Typically the channels may extend across the front and back half of the channels, meaning that they cross the transversal centerline 90. The back half of the article is defined as the area of the article between the back edge 12 of the article and the transversal centerline 90. The front half of the article is the area of the article between the front edge 10 and the transversal centerline 90.

According to the invention, the intermediate layer also comprises a back pocket 87 which is at least partially, and typically entirely disposed on the back half of the article. The back pocket is also substantially free of fibrous material and provides a space for viscous or runny bowel movements to be stored and separated from the skin of the wearer. There is therefore less chance for these exudates to spread out of the diaper and leak. The back pocket is at least partially and typically entirely located in the back half the absorbent article in order to directly receive the bowel movement. The back pocket has a length d3 in the longitudinal direction. The length d3 of the back pocket may typically be at least about 5%, in particular at least about 10% of the length L of the article, in particular from about 8% to about 40%, more particularly from about 10% to 30%, of the length L of the article. The channels may have a longer length d2 than the length of the back pocket d3 as represented in the Figures, but it is not excluded that these lengths may be also about equal, or that the back pocket length d3 may be longer than the length of the channels d2. In order to measure the respective lengths, the transition between the channels and the back pocket is taken at the point where the fibrous central area 82 between the channels stops, as shown in Fig. 5.

According to the present invention, the back pocket 87 is connected to the first and second channels 86, so that these together form a unitary connected area 86-87 in the intermediate layer which is substantially free of the fibrous material. By connecting the back pocket to the channels, it was found that the diaper could more easily assume a three dimensional basin shape when worn by the wearer. It was found in particular that by connecting the channels to the back pocket, the channels could more efficiently function as hinges and improve the flexibility of the diapers along the channels. In this basin shape, the lateral areas of the diapers outwards of the channels can form upwards barriers that can help maintaining a larger void between the topsheet and the skin of the wearer. This larger void can contain the runny or pasty bowel movements in the back pocket. This was found to maximize the space available for the runny waste while keeping enough fibrous material in the intermediate layer to efficiently distribute other fluid insults to the underlying core. This overall provides better barrier protection against leakage from the intermediate layer.

The intermediate layer typically also comprises a front area 84 having a length d1 in the longitudinal direction disposed longitudinally in front of the channels 86. The front area starts where the channels extremities end, as shown on Fig. 5. The intermediate layer advantageously also comprises a back area 85 having a length d4 disposed longitudinally backward of the pocket 87. The intermediate layer also advantageously comprises lateral areas 81 and 83 comprising fibrous material and disposed laterally outwardly of the unitary area formed by the combined channels and pocket. The lateral areas may thus have the same length d2+d3 as the combined unitary area. The channels and back pocket advantageously do not extend up to any of the edges of the intermediate layer, and are therefore surrounded by and fully encompassed within the intermediate layer by the front, back and lateral areas of the intermediate layer which all comprise fibrous material. The smallest distance between the unitary area 86-87 and the closest outer edge of the intermediate layer may for example be at least 5 mm, or at least 10 mm.

The overall surface of the substantially material-free channels and back pocket combined unitary area may typically represent from 5% to 50% of the overall surface of the intermediate layer, in particular from 10% to 40%. In the example shown in Figure 5, the surface of the channels and back pocket is about 23% of the overall surface of the intermediate layer. Exemplary dimensions for a size 2 diaper are d1=53 mm, d2=100 mm, d3=50 mm, d4=40mm and L = 400 mm. The back pocket may be about equal or larger in surface than the combined surface of both channels, for example the surface of the back pocket may range from about 100% to about 150% of the surface of both channels combined. It is also not excluded that the surface of the back pocket may be less than the combined surface of both channels.

The channels may be typically at least partially curved. In particular the channels may be concave (inwardly curved) towards the longitudinal axis 80 as illustrated in the Figures. Alternatively, it is not excluded that the channels may be partially or entirely straight, and in particular longitudinally oriented parallel to the longitudinal axis 80, or curved in the other direction. The channels are typically disposed as one or more symmetrical pair(s) relative to the longitudinal axis, and are spaced apart from one another over their whole longitudinal dimension. The width of the channels may remain about constant along most of their length as shown, or their width may vary along their length, for example the widths of the channels may increase from the back to the front of the article. The width of the channels may typically range from about 2 mm to about 20 mm, in particular from about 4 mm to about 16 mm, for example from about 8 to about 10 mm, at least for a portion of their length.

The general outer shape of the intermediate layer as seen from above may be shaped (that is non-rectangular), for example having a bullet shape as illustrated on Fig. 5 with a back edge that is outwardly rounded, but it may also be generally rectangular as illustrated in Fig. 6, as is typical in the art. The different configurations can be used to maximize the efficiency of the intermediate layer for different applications. When the back edge of the intermediate layer is outwardly curved (bulging out towards the back of the article) as in Fig. 5, this removes some fibrous material at least in the back corner areas of the intermediate layer, which are the areas less likely to be contacted by an insulting fluid and thus represent an efficient material savings.

The intermediate layer may also have a profiled basis weight configuration in the longitudinal and/or transversal direction. The average basis weight of the central area 82 may for example differ from the average basis weight in the lateral areas 81,83 by at least 30 g/m$^2$ (grams per square meter, or gsm), in particular at least 40 g/m$^2$, or at least 50 g/m$^2$, or at least 60 g/m$^2$. The central area 82 and/or the front area 84 may have a higher average basis weight relative to the first and second lateral areas 81, 83 and/or the back area 85. The average basis in any of the areas may typically range from 50 g/m$^2$ and 350 g/m$^2$, in particularly from 100 g/m$^2$ to 300 g/m$^2$ but other values are possible depending of the type of absorbent articles and fibrous material considered. For some articles like adult incontinence products having to deal with relatively high amount of fluid, these values may be higher, whereas articles for small babies may require less.

The average basis weight in each area of the intermediate layer can be measured experimentally by removing and weighing the fibrous material in the area considered (central area, both lateral areas, or back area) and dividing this weight by the surface of the area considered. The surface of the area can be determined by taking a picture and analyzing it using any commercial image analysis software, or by any other conventional methods. The channels and any other areas which are substantially free of fibrous material are disregarded for calculating the average basis weight of the different areas. It is unlikely that the average basis weights in the first lateral area and the second lateral area substantially differs. In any case, the combined weight of the material in both areas and the combined surface of both lateral areas is used to determine the average basis weight of the lateral areas.

Independently of the relation between the basis weight of the central and the lateral areas, it may be further advantageous that the basis weight of the fibrous material of the intermediate layer measured at the P point (P) is maximum relative to the rest of the intermediate layer. By maximum, it is meant that the intermediate layer does not comprise another point having a higher basis weight, but of course there may be other point or regions in the intermediate layer having the same basis weight. The P point is defined as the point of the intermediate layer on the longitudinal axis disposed at a distance of 0.30 of the length of the article (L) from the front edge 10 of the article. The basis weight at the P point, or on any the other regions of the intermediate layer, can be typically directly determined from the manufacturer's specification of the layer making machine. If the specification is not known, the local basis weight at the P point may be measured by taking a circular sample having a diameter of 10 mm and centered on the P point. The material in this circular sample area is weighed and divided by the surface area of the circle to provide the local basis weight value.

Although not explicitly represented in the Figures, the topsheet 24 and/or the acquisition layer 52 if present may be attached to the top side of the core cover 16 through the channels and/or the back pocket of the intermediate layer. These layers may be bonded for example with an adhesive, see WO2015/031243 (Roe et al.) for example of such attachments.

As indicated previously, the intermediate layer is comprised of a fibrous material. The intermediate layer may be a nonwoven material comprising fibers that are bonded to another by any known means so that the layer has a strong integrity and may be manipulated independently of a substrate. A suitable nonwoven may be for example a high loft carded nonwoven as is known in the art. The channels and back pocket may be formed in such a nonwoven by cutting out the desired areas.

Instead of a nonwoven, the intermediate layer may also comprise or consist of loose synthetic and/or cellulose fibers with no or weak intra-fiber bonds. These fibers may be typically deposited on a supporting substrate, optionally at varying basis weight to form a profiled distribution. A typical example of such fibrous material comprises or consists of cross-linked cellulose fibers. The intermediate layer may for example comprise at least 50% by weight of cross-linked cellulosic fibers. The cross-linked cellulosic fibers may be crimped, twisted, or curled, or a combination thereof including crimped, twisted, and curled. This type of material has been used in the past in disposable diapers as part of an acquisition and distribution system, for example US 2008/312622 A1 (Hundorf et al.). The cross-linked cellulosic fibers provide higher resilience and therefore higher resistance against the compression in the product packaging or in use conditions, e.g. under baby weight.

Exemplary chemically cross-linked cellulosic fibers suitable for an intermediate layer are disclosed in US5,549,791, US5,137,537, WO95/34329 or US2007/118087. Exemplary cross-linking agents include polycarboxylic acids such as citric acid and/or polyacrylic acids such as acrylic acid and maleic acid copolymers. For example, the cross-linked cellulosic fibers may have between about 0.5 mole % and about 10.0 mole % of a C2-C9 polycarboxylic acid cross-linking agent, calculated on a cellulose anhydroglucose molar basis, reacted with said fibers in an intrafiber ester crosslink bond form. The C2-C9 polycarboxylic acid cross-linking agent may be selected from the group consisting of:

- aliphatic and alicyclic C2-C9 polycarboxylic acids having at least three carboxyl groups per molecule; and
- aliphatic and alicyclic C2-C9 polycarboxylic acids having two carboxyl groups per molecule and having a carbon-carbon double bond located alpha, beta to one or both of the carboxyl groups, wherein one carboxyl group in said C2-C9 polycarboxylic acid cross-linking agent is separated from a second carboxyl group by either two or three carbon atoms. The fibers may have in particular between about 1.5 mole % and about 6.0 mole % cross-linking agent, calculated on a cellulose anhydroglucose molar basis, reacted therewith in the form of intrafiber ester crosslink bonds. The cross-linking agent may be selected from the group consisting of citric acid, 1, 2, 3, 4 butane tetracarboxylic acid, and 1, 2, 3 propane tricarboxylic acid, in particular citric acid.

Polyacrylic acid cross-linking agents may also be selected from polyacrylic acid homopolymers, copolymers of acrylic acid, and mixtures thereof. The fibers may have between 1.0 weight % and 10.0 weight %, preferably between 3 weight % and 7 weight %, of these cross-linking agents, calculated on a dry fiber weight basis, reacted therewith in the form of intra-fiber crosslink bonds. The cross-linking agent may be a polyacrylic acid polymer having a molecular weight of from 500 to 40,000, preferably from 1,000 to 20,000. The polymeric polyacrylic acid cross-linking agent may be a copolymer of acrylic acid and maleic acid, in particular wherein the weight ratio of acrylic acid to maleic acid is from 10:1 to 1:1, preferably from 5:1 to 1.5:1. An effective amount of citric acid may be further mixed with said polymeric polyacrylic acid cross-linking agent.

The intermediate layer comprising cross-linked cellulose fibers may comprise other fibers, but this layer may comprise at least 50%, or 60%, or 70%, or 80%, or 90% or even up to 100%, by weight of the layer, of cross-linked cellulose fibers (including the cross-linking agents). Examples of such mixed layer of cross-linked cellulose fibers may comprise about 70% by weight of chemically cross-linked cellulose fibers, about 10% by weight polyester (PET) fibers, and about 20 % by weight untreated pulp fibers. In another example, the layer of cross-linked cellulose fibers may comprise about 70% by weight chemically cross-linked cellulose fibers, about 20% by weight lyocell fibers, and about 10% by weight PET fibers. In another example, the layer may comprise about 68% by weight chemically cross-linked cellulose fibers, about 16% by weight untreated pulp fibers, and about 16% by weight PET fibers. In another example, the layer of cross-linked cellulose fibers may comprise from about 90-100% by weight chemically cross-linked cellulose fibers.

The fibrous intermediate layer may be manufactured by air-laying the fibers on a drum having the required profile. A plurality of such molds may be arranged in a repeating manner along the circumference of a drum in an air-laying chamber, as is known in the art. The bottom of the mold may comprise a grid connected to a source of vacuum so that the fibers are pulled and deposited in the mold. Two elongated protuberances for the channels connected to a pocket-shaped protuberance are flush with the frame of the receptacle, so that substantially no fibers deposit in these areas to form the two channels and back pocket of the intermediate layer. The mold may further comprise zones of different depths, to provide a profiled basis weight distribution, if desired. The formed intermediate layer can then be directly unmolded onto another component of the article such as a nonwoven and then integrated with the rest of the chassis of the article. When a nonwoven acquisition layer 52 is present in the article, the intermediate layer may be for example deposited on this acquisition layer, the two layers being further joined to absorbent core and the rest of the article, as is known in the art. Alternatively, the fibers may be deposited directly on another layer such as the topsheet if no acquisition layer is present.

Some of the possible other components of an absorbent article will be further discussed and exemplified below. The following description should not be considered limiting the claims unless otherwise specified.

## Topsheet 24

The topsheet typically forms the majority of the wearer-contacting surface of the article and is the first layer that the body exudates contact. The topsheet is preferably compliant, soft-feeling, and non-irritating to the wearer's skin. Further, at least a portion of the topsheet is liquid permeable, permitting liquids to readily penetrate through its thickness. Any known topsheet may be used in the present invention, however the invention may be particularly useful when the topsheet comprises a plurality of apertures to ease penetration of exudates therethrough, such as urine and/or feces (solid, semi-solid, or liquid). This type of apertured topsheet is for example used in the Pamper® diapers line-up for new babies. The apertures are typically elongated in the longitudinal direction and from about 2 to about 6 mm long with an aspect ratio ranging for example from about 1:2 to about 1:5. Apertured topsheets may in particular have a percentage of openness that ranges from about 7% to 30% of the overall surface of the topsheet. Apertured topsheets are for example disclosed in US6,632,504 (Gillespie et al.).

The topsheet may also comprise a single larger macro-aperture as has been proposed in the past. The size of this primary aperture is important in achieving the desired waste encapsulation performance. If the primary aperture is too small, the waste may not pass through the aperture, either due to poor alignment of the waste source and the aperture location or due to fecal masses having a diameter greater than the aperture. If the aperture is too large, the area of skin that may be contaminated by "rewet" from the article is increased. Typically, the total area of such macro-apertures at the surface of a diaper may range from about 10 cm$^2$ to about 50 cm$^2$, in particular from about 15 cm$^2$ to about 35 cm$^2$. The macro-aperture may in particular be disposed at least partially over the back pocket of the intermediate layer.

On the other hand, the present invention may be used with other conventional topsheets and suitable topsheets may be manufactured from a wide range of materials. Most topsheets are nonwoven materials or apertured formed films. Typical diaper topsheets have a basis weight of from about 10 gsm to about 28 gsm, in particular between from about 12 gsm to about 18 gsm but higher basis weights are possible if it is desired to provide a very soft feeling wearer-contacting surface for example.

Nonwoven topsheets may be made of natural fibers (e.g., wood or cotton fibers), synthetic fibers or filaments (e.g. polyester or polypropylene or bicomponent PE/PP fibers or mixtures thereof), or a combination of natural and synthetic fibers. If the topsheet includes nonwoven fibers, the fibers may be spunbond, carded, wet-laid, meltblown, hydroentangled, or otherwise processed as is known in the art. In particular the topsheet may be a spunbond PP nonwoven or a through-air bonded carded nonwoven web ("TABCW"). Nonwoven topsheets may comprise fusion bonds as is known in the art. WO 2011/163582 also discloses suitable colored topsheet having a basis weight of from 12 to 18 gsm and comprising a plurality of bonded points. Each of the bonded points has a surface area of from 2 mm$^2$ to 5 mm$^2$ and the cumulated surface area of the plurality of bonded points is from 10 to 25% of the total surface area of the topsheet.

Apertured films may also be used as topsheet. Suitable formed film topsheets are for example described in US3,929,135, US4,324,246, US4,342,314, US4,463,045, and US5,006,394. Other suitable apertured film topsheets may be made in accordance with US4,609,518 and US4,629,643.

The topsheet may also have a three-dimensional appearance and feel, or there may be an additional, smaller, three-dimensional layer placed on top of the topsheet. Such three-dimensional additional layers may be for example particularly useful to receive low viscous exudates such as the stool of young babies. Examples of such fluid entangled dual layered three-dimensional materials and processes to obtain them have been disclosed for example in US2014/0121623A1, US2014/0121621A1, US2014/0121624A1, US2014/0121625A1.

The topsheet may be treated with a wetting agent to make it more hydrophilic. The wetting agent may be a surfactant as is known in the art. Other possible treatments are for example special coating by nanoparticles, as for example described in US6,645,569, US6,863,933, US2003/148684 and US2005/008839 (Cramer et al.) and US7,112,621 (Rohrbaugh et al). Any portion of the topsheet may also coated with a lotion as is known in the art. Examples of suitable lotions include those described in US5,607,760, US 5,609,587, US5,643,588, US5,968,025 and US6,716,441. The topsheet may also include or be treated with antibacterial agents, some examples of which are disclosed in WO95/24173. Further, the topsheet, the backsheet or any portion of the topsheet or backsheet may be embossed and/or matte finished to provide a more cloth like appearance.

The topsheet may be typically attached to the underlying layer such as a nonwoven acquisition layer 52 by gluing over its whole area or over a portion thereof. Apertured topsheets may be typically only partially attached to the underlying layer to ease the penetration of the runny / pasty bowel movement to the underlying layer. The topsheet may be in particular not or only partially attached to the underlying layer in the area corresponding to the channels and /or the back pocket. However it is not excluded that an apertured topsheet or any other topsheets may be attached over their whole surface to the underlying layer.

**Acquisition layer 52**

The absorbent article may optionally comprise an acquisition layer 52 in addition to the intermediate layer 54. An acquisition layer can quickly pull the fluid away from the topsheet so as to provide a good dryness feeling for the wearer. The acquisition layer is typically placed directly under the topsheet so that the intermediate layer may be then at least partially disposed under the acquisition layer. The acquisition layer may for example be used as a support for the fibers of the intermediate layer during the manufacturing process. The acquisition layer may typically be or comprise a nonwoven material, for example a SMS or SMMS material, comprising a spunbonded, a melt-blown and a further spunbonded layer or alternatively a carded chemical-bonded nonwoven. Exemplary acquisition layers are disclosed in US7,786,341. Carded, resin-bonded nonwovens may be used, in particular where the fibers used are solid round or round and hollow PET staple fibers (50/50 or 40/60 mix of 6 denier and 9 denier fibers). The acquisition layer may be stabilized by a latex binder, for example a styrene-butadiene latex binder (SB latex). Nonwovens have the advantage that they can be manufactured outside the converting line and stored and used as a roll of material. Further useful nonwovens are described in US6,645,569 (Cramer et al.), US6,863,933 (Cramer et al.), US7,112,621 (Rohrbaugh et al.), US2003/148684 (Cramer et al.) and US2005/008839 (Cramer et al.). Processes for obtaining such latices are known, for example, from EP149880 (Kwok) and US 2003/0105190 (Diehl et al.). The binder may typically be present in the acquisition layer in amount ranging from about 12% to about 50%, for example about 30%, by total weight of the acquisition layer. SB latex is available under the trade name GENFLO™ 3160 (OMNOVA Solutions Inc.; Akron, Ohio).

Another typical acquisition layer, sometimes referred to as secondary topsheet, may for example be a through-air bonded carded web ("TABCW") but many other alternatives material are known in the art and may be used instead. "Bonded carded web" refers to webs that are made from staple fibers that are sent through a combing or carding unit, which breaks apart and aligns the staple fibers in the machine direction to form a generally machine direction-oriented fibrous nonwoven web. This web is then drawn through a heated drum, creating bonds throughout the fabric without applying specific pressure (thru air bonding process). The TABCW material provides a low density, lofty through-air bonded carded web. The web may for example have a specific weight basis level at about 15 gsm to about 120 gsm (gram per m$^2$), in particular about 30 gsm to about 80 gsm. The TABCW material can for example comprise about 3 to about 10 denier staple fibers. Examples of such TABCW are disclosed in WO2000/71067 (KIM DOO-HONG et al.). TABCW are available directly from all usual suppliers of nonwoven webs for use in absorbent articles, for example Fitesa Ltd or Fiberweb Technical Nonwovens.

A further acquisition layer (not shown) may be used in addition to the first acquisition layer described above. For example a tissue layer may be placed between the first acquisition layer and the intermediate layer. The tissue may have enhanced capillarity distribution properties compared to the acquisition layers described above. The tissue and the first acquisition layer may be of the same size or may be of different size, for example the tissue layer may extend further in the back of the absorbent article than the first acquisition layer. An example of a hydrophilic tissue is a 13 to 15 gsm high wet strength tissue made of cellulose fibers from supplier Havix.

**Absorbent core 28**

The absorbent article comprises an absorbent core 28 which comprises an absorbent material 60 for acquiring and storing fluid. The absorbent cores of the invention may be any conventional absorbent cores known in the art. The absorbent core typically comprises an absorbent material contained in a core wrap. As used herein, the term "absorbent core" does not include the topsheet, the backsheet, and the intermediate layer. The absorbent core has typically the most absorbent capacity of all the components of the absorbent article, and comprises all or at least the majority of superabsorbent polymer (SAP) in the article. The core typically may thus consist essentially of, or consists of, the core wrap, the absorbent material and optionally adhesives. The absorbent material may consist of SAP in particulate form as exemplified in the present description but it is not excluded that other type of absorbent material may be used. The terms "absorbent core" and "core" are herein used interchangeably.

The absorbent material in the core may typically comprise fibers mixed with superabsorbent polymer particles. The fibers may typically comprise wood pulp (cellulose) fibers optionally mixed with synthetic fibers. The absorbent material typically comprises from 50% to 90% of superabsorbent polymers (herein abbreviated as "SAP" also referred to as absorbent gelling material) by weight of the absorbent material. The absorbent material may for example comprise at least 55% superabsorbent polymers by weight of the absorbent material, in particular from 60% to 90% superabsorbent polymers by weight of the absorbent material, in particular from 65% to 85% superabsorbent polymers by weight of the absorbent material. It is not excluded that higher amount of SAP may be present, and in some cases it may be possible that the absorbent material comprise little or no cellulose fibers (so called airfelt-free cores).

In airfelt-free cores, the absorbent material may be deposited within the core wrap as one layer, (continuously or in discrete pockets) or as two absorbent layers applied on the top substrate 16 and bottom substrate 16' respectively in a pattern of land areas separated by junction areas. The two absorbent layers having offset land areas and junction areas may be combined to form an absorbent material deposition area in which the absorbent material is substantially continuous. This dual layer construction is for example generally disclosed in WO2008/155699 (Hundorf et al.). Airfelt-free absorbent cores may also comprise a fibrous thermoplastic adhesive to further immobilize the absorbent material. The SAP printing technology as disclosed for example in US2006/024433 (Blessing), US2008/0312617 and US2010/0051166A1 (both to Hundorf et al.) may be used. This technique uses a transfer device such as a printing roll to deposit SAP onto a substrate disposed on a grid of a support which may include a plurality of crossbars extending substantially parallel to each other and spaced apart from one another. This technology allows high-speed and precise deposition of SAP on a substrate in particular to provide one or more area(s) substantially free of absorbent material surrounded by absorbent material. Channel areas 26 substantially free of absorbent material can be formed for example by modifying the pattern of the grid and receiving drums so that no SAP is applied in the selected areas, as exemplary disclosed in US2012/0312491 (Jackels). The absorbent cores of the present invention are however not limited to a particular process for making them, and the cores of the invention may be more conventionally made by air-laying a mix of cellulose fibers and superabsorbent particles on a conventional air-laying drum.

The absorbent core can notionally (i.e. virtually) comprise a longitudinal axis extending from its front edge to the back edge and dividing the core in two substantially symmetrical halves relative to this axis, when viewing the core in the plane formed by the longitudinal and transversal direction. The longitudinal axis of the core may be generally parallel and superposed with the longitudinal axis of the absorbent article. The absorbent core can typically be generally rectangular including the region of the core wrap which does not enclose the absorbent material, in particular at the front and back end seals when present. In case the core is not rectangular, the maximum dimension measured along the transversal direction and the longitudinal direction can be used to report the width and length of the core respectively. The width and length of the core may vary depending on the intended usage. For baby and infant diapers, the width of the core may for example in the range from 40 mm to 200 mm and the length of the core from 100 mm to 600 mm. Adult incontinence products may have higher maximum dimensions.

The absorbent core may comprise any conventional absorbent materials used in absorbent articles. The absorbent material may consist of SAP particles immobilized by an adhesive to provide a relatively thin core. The absorbent core may thus be relatively thin, in particular thinner than conventional cores comprising cellulosic fibers. In particular, the caliper of the core (dry, i.e. before use) as measured at the center point (C) of the article or at any other points of the surface of the core according to the Dry Core Caliper Test as described herein may be from 0.25 mm to 5.0 mm, in particular from 0.5 mm to 4.0 mm. However other types of absorbent material are more commonly used, the absorbent material may be in particular a mix of cellulose fibers and SAP particles.

## Channel areas 26 in the absorbent core

The intermediate layer may advantageously cooperate with the underlying absorbent core to provide improved performance in terms of fluid handling and/or wearing comfort. The absorbent core 28 may in particular advantageously comprise at least two longitudinally-extending channel areas 26a, 26b. The channel areas 26 may be longer or shorter than the channels 86 of the intermediate layer, but advantageously the channel areas of the core correspond at least along a portion of their length to the channels in the intermediate layer. In this way, the fluid can be directly transferred vertically first via the channels in the intermediate layer to the channel areas of the absorbent core, where it can be spread longitudinally along the length of the channels. The absorbent core may also comprise a rear pocket that may be at least partially superposed with the back pocket of the intermediate layer. Exemplary absorbent cores comprising channel areas and rear pockets are disclosed for example in WO2014/093129A1 (Roe et al.).

The channel areas may be substantially free of absorbent material, and core wrap bonds 27 may be formed through these channels areas. By "substantially free" it is meant that zones do not comprise absorbent material except possibly for minimal amount such as involuntary contaminations with absorbent material particles that may occur during the core making process. The top side 16 of the core wrap may be attached to the bottom side 16' of the core wrap by core wrap bonds 27 through these areas substantially free of absorbent material. The channel areas 26 are advantageously surrounded by absorbent material 60. When the absorbent material swells upon absorbing a liquid, the core wrap bonds 27 remain at least initially attached in the channel areas 26. The absorbent material 60 swells in the rest of the core when it absorbs a liquid, so that the core wrap forms one or more visible channels along the channel areas 26 comprising the core wrap bond. These channels are three dimensional and can serve to distribute an insulting fluid along their length to a wider area of the core. They may provide a quicker fluid acquisition speed and a better utilization of the absorbent capacity of the core. It is not excluded that the channel areas may be without a core wrap bond, as this may be easier to manufacture, but these non-bonded areas will typically not form permanent three-dimensional channels when wet.

The top side and the bottom side of the core wrap may be bonded together continuously along the channel areas 26, but the core wrap bond 27 may also be discontinuous (intermittent) such as formed by series of point bonds. An auxiliary glue 72 may be used to at least partially form the substrates bond 27. In this case, some pressure may be applied on the substrates in the zones 26 to improve the adhesive bonds between the substrates. If an optional fibrous adhesive is present, it may also help forming the bond 27. If the auxiliary glue is applied as a series of longitudinally oriented continuous slots, the width and frequency of these slots may advantageously be such that at least one slot of auxiliary glue is present at any level of the channel area 26 in the longitudinal direction. For example the slots may be 1 mm wide with a 1 mm distance between each slots, and the channel areas have a width of about 8 mm. Such on average for 4 slots of auxiliary glue will be present in each of the channel area 26. It is of course also possible to form the bonds 27 via other known attachment means, such as pressure bonding, ultrasonic bonding, heat bonding or combination thereof.

The channel areas 26 extend substantially longitudinally, meaning that each zone extends at least as much in the longitudinal direction than in the transversal direction, and typically at least twice as much in the longitudinal direction than in the transverse direction (as measured after projection on the respective axis). The channel areas 26 may have a length projected on the longitudinal axis 80 of the core that is at least 10% of the length L of the absorbent article, in particular from 15% to 70%. The channel areas may have a width along at least part of their length which is at least 2 mm, or at least 3 mm or at least 4 mm, up to for example 20 mm, or 16 mm or 12 mm. The width of each channel areas substantially free of absorbent material may be constant through substantially its whole length or may vary along its length.

The channel areas may be typically at least partially curved. In particular the channel areas present in the crotch region may be concave towards the longitudinal axis 80 as illustrated in the Figure. Alternatively, it is not excluded that the channel areas may be partially or entirely straight, and in particular longitudinally oriented parallel to the longitudinal axis 80, or curved in the other direction. The channel areas are typically disposed as one or more symmetrical pair(s) relative to the longitudinal axis, and are spaced apart from one another over their whole longitudinal dimension.

Typically the absorbent core may be longer and wider than the intermediate layer, so that the channel areas 26 can extend further than the channels 86 of the intermediate layer. It may be advantageous that the channels 86 or channel areas 26 do not reach any of the edges of the layer in which they are formed, to reduce the risk of fluid escaping the layer. Thus the channels and channel areas may be designed to stop at a distance of at least 5 mm from any edges of the layer in which they are formed. It is however also not excluded that the intermediate layer may be as wide and/or as long as the absorbent core, and the channels and the channel areas may have similar dimensions. It is also not excluded that the channel areas 26 of the core when present are not superposed with the channels 86 of the intermediate layer 54.

The absorbent core may have a profiled distribution of absorbent material in the longitudinal direction, especially having a higher basis weight in the front half of the article than in the back half of the article. The absorbent core may also be profiled in the transversal direction, in particular, the absorbent core and the intermediate layer may be similarly profiled in the transversal direction. Thus, the average basis weight of the absorbent material in the central absorbent zone of the core (the zone between the channel areas) between the channel areas 26 may also differ (typically higher but it may also be lower) than the average basis weight in the first and second lateral absorbent zones of the core disposed outwardly of the channel areas and having the same length. The average basis weight of the absorbent material in the central absorbent zone may differ by at least 10% than the average basis weight in the first and second lateral absorbent zones, in particular by at least 25%, or by at least 50%, or by at least 100%. The absorbent core may also comprise a back pocket substantially free of absorbent material that may or may not be connected to the channel areas. This back pocket may be generally aligned with the back pocket in the intermediate layer.

The total amount of absorbent material should be sufficient for the application considered. For baby diapers for example, the amount of superabsorbent material (SAP) should be sufficient to provide overnight dryness. For children having a weight range of 8-17 kg, as an example, the total amount of SAP in the core may range from about 8 g to about 12 g or more. The present invention is especially useful for smaller size diapers for new babies, and in these cases the overall amount of SAP in the article may for example range from about 2 g to about 10 g, in particular from about 3 g to about 8 g of SAP.

**Core wrap 16, 16'**

The absorbent core typically comprises a core wrap which encloses the absorbent material. Various core wrap constructions are possible. The core wrap may in particular comprise as represented in the Figures two separate substrates 16, 16' forming the top side and the bottom side of the core wrap respectively. The two substrates may be attached in a C-wrap configuration with one of the substrate larger than the other so that it can form two flaps that are folded back on the other substrate, the flaps are then bonded by two longitudinal seals 284', 286'. The core wrap may also optionally comprise a front edge seal and a back edge seal. However this core wrap construction is not limiting of the invention, as any conventional core wrap construction may also be used, for example a single substrate on a portion of which the absorbent material is deposited and then the rest of the substrate folded over the deposited absorbent material to form the other side of the core. This single substrate construction can then be sealed longitudinally with a single longitudinal edge seal. The core wrap may also comprise two substrates disposed flat in a face to face relation (sandwich).

The substrates may be formed by any materials suitable for receiving and containing the absorbent material. Typical substrate materials used in the production of conventional cores may be used, in particular paper, tissues, films, wovens or nonwovens, or laminate of any of these. As used herein, the terms "nonwoven layer" or "nonwoven web" generally means an engineered fibrous assembly which has been given a designed level of structural integrity by physical and/or chemical means with the exclusion of paper, woven or knitted materials. The fibers may be of natural or synthetic origin and may be staple or continuous filaments or be formed in situ. Nonwoven webs can be formed by many processes such as meltblowing, spunbonding, solvent spinning, electrospinning, carding and airlaying. The basis weight of nonwoven webs is usually expressed in grams per square meter (g/m$^2$ or gsm). Nonwoven materials are typically made of synthetic fibers, such as PE, PET and in particular PP fibers.

The core wrap may in particular be formed by a nonwoven web, such as a carded nonwoven, spunbond nonwoven ("S") or meltblown nonwoven ("M"), and laminates of any of these. For example spunmelt polypropylene nonwovens are suitable, in particular those having a laminate web SMS, or SMMS, or SSMMS, structure, and having a basis weight range of about 5 gsm to 15 gsm. Suitable materials are for example disclosed in US 7,744,576, US 2011/0268932A1, US2011/0319848A1 and US2011/0250413A1. It is also possible than the core wrap may be at least partially formed from a component of the article having another function than substrate for the absorbent material. For example, it is possible that the backsheet may form the bottom side of the core wrap and/or that an intermediate layer or the topsheet may form the top side of the core wrap. However, typically the core wrap is made of one or more substrates whose only function is to receive and enclose the absorbent material, as indicated previously.

**Absorbent material 60**

The absorbent material may be any known absorbent material known in the art, but will typically comprise or consist of superabsorbent polymers (herein referred to as "SAP"). The SAP may be typically in particulate forms (superabsorbent polymer particles), optionally mixed with cellulose fibers, but it not excluded that other forms of SAP may be used such as a superabsorbent polymer foam for example. The SAP useful in the present invention includes a variety of water-insoluble, but water-swellable polymers capable of absorbing large quantities of fluids. The term "superabsorbent polymer" refers herein to absorbent materials, which may be cross-linked polymeric materials, that can typically absorb at least 10 times their weight of an aqueous 0.9% saline solution as measured using the Centrifuge Retention Capacity (CRC) test (EDANA method WSP 241.2.R3 (12). The SAP may in particular have a CRC value of more than 20 g/g, or more than 24 g/g, or of from 20 to 50 g/g, or from 20 to 40 g/g, or 24 to 30 g/g.

The superabsorbent polymers may be in particulate form so as to be flowable in the dry state and thus easily deposited on a substrate. Typical particulate absorbent polymer materials are made of poly(meth)acrylic acid polymers. However, starch-based particulate absorbent polymer materials may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile. The superabsorbent polymer may be polyacrylates and polyacrylic acid polymers that are internally and/or surface cross-linked. Suitable materials are described in WO07/047598, WO07/046052, WO2009/155265 and WO 2009/155264. Suitable superabsorbent polymer particles may be obtained by current state of the art production processes, for example as described in WO2006/083584. The superabsorbent polymers are preferably internally cross-linked, i.e. the polymerization is carried out in the presence of compounds having two or more polymerizable groups which can be free-radically copolymerized into the polymer network.

The absorbent core typically comprises only one type of SAP, but it is not excluded that a blend of different SAPs may be used. The fluid permeability of a superabsorbent polymer can be quantified using its Urine Permeability Measurement (UPM) value, as measured in the test disclosed in US patent application number US2014/005622A1. The UPM of the SAP may for example be of at least 10 x10-7 cm3.sec/g, or at least 30 x10-7 cm3.sec/g, or at least 50 x10-7 cm3.sec/g, or more, e.g. at least 80 or 100 x10-7 cm3.sec/g. The SAP particles may have a time to reach an uptake of 20 g/g (T20) of less than 240s, preferably from 40s to less than 240s, more preferably from 65s to 215s, as measured according to the K(t) test method as described in WO2015/041784 (Peri et al).

**Fastening system 42, 44**

The absorbent article may include a fastening system, especially when the article is a taped diaper as exemplified in Fig. 1. The fastening system can be used to provide lateral tensions about the circumference of the absorbent article to hold the absorbent article on the wearer. Such a fastening system is not necessary for pant articles such as baby diaper pants, training pants and adult incontinence pants since the waist region of these articles is already bonded and elasticized. The fastening system usually comprises a fastener 42 such as tape tabs, hook and loop fastening components, interlocking fasteners such as tabs & slots, buckles, buttons, snaps, and/or hermaphroditic fastening components, although any other known fastening means are generally acceptable. A landing zone 44 is normally provided on the front waist region of the article for the fastener 42 to be releasably attached. Some exemplary surface fastening systems are disclosed in US3,848,594, US4,662,875, US4,846,815, US4,894,060, US4,946,527, US5,151,092 and US5,221,274 (Buell). An exemplary interlocking fastening system is disclosed in US 6,432,098. The fastening system may also provide a means for holding the article in a disposal configuration as disclosed in US4,963,140 (Robertson et al.)

The fastening system may also include primary and secondary fastening systems, as disclosed in US 4,699,622 to reduce shifting of overlapped portions or to improve fit as disclosed in US 5,242,436, US 5,499,978, US 5,507,86, and US 5,591,152.

**Backsheet 25**

The backsheet may be any backsheet known in the art for absorbent articles. The backsheet may be positioned directly adjacent the garment-facing surface of the absorbent core. The backsheet prevents, or at least inhibits, the exudates absorbed and contained therein from soiling articles such as bedsheets and undergarments. The backsheet is typically impermeable, or at least substantially impermeable, to liquids (e.g., urine). The backsheet may, for example, be or comprise a thin plastic film such as a thermoplastic film having a thickness of less than 0.1 mm. The basis weight of those films is usually as low as possible to save material costs, typically from 10 gsm to 30 gsm, in particular below 20 gsm. A covering low basis weight nonwoven may be attached to the external surface of the film to provide for a softer touch.

Suitable backsheet materials include breathable materials which permit vapors to escape from the absorbent article while still preventing, or at least inhibiting, exudates from passing through the backsheet. Example breathable materials may include materials such as woven webs, nonwoven webs, composite materials such as film-coated nonwoven webs, microporous films. The film may include at least about 20 weight percent filler particles, for example filler particles that include calcium carbonate, so that wherein the film has been stretched in the machine direction, e.g. to at least about 150 percent, fractures are formed where said filler particles are located. The films may be biaxially stretched at least about 150 percent in the machine direction and a transverse direction to cause fractures to form where said filler particles are located. Breathable films may generally have Water Vapor Transmission Rates (WVTR) in excess of 300 grams per square meter per 24 hours. The WVTR may be measured by the Desiccant Method as indicated in ASTM E96/E96M - 14.

The backsheet may further typically comprise a nonwoven on its most external side to improve softness. Exemplary laminates comprising a breathable film and a nonwoven layer are for example disclosed in WO2014/022,362A1, WO2014/022,652A1 and US5,837,352. The nonwoven web may in particular comprise a spunbond nonwoven web and/or a laminate of a spunbond nonwoven web and a meltblown nonwoven web. The laminate may also have a water vapor transmission rate of at least 300 g/m2/24 hours. US5,843,056 for example discloses substantially liquid impermeable, vapor permeable composite backsheet.

**Front and back ears 46, 40**

The absorbent article may comprise front ears 46 and back ears 40 as is known in the art for taped diapers. Diaper pants which are already sealed along the waist edges typically do not require front ears and back ears. The ears can be integral part of the chassis, for example formed from the topsheet and/or backsheet as side panel. Alternatively, as represented in Fig. 1, they may be separate elements attached by gluing and/or heat embossing. The back ears 40 are optionally stretchable to facilitate the attachment of the tabs 42 on the landing zone 44 and maintain the taped diapers in place around the wearer's waist. The front ears 46 may also be optionally elastic or extensible to provide a more comfortable and contouring fit.

**Barrier leg cuffs 34 and gasketing cuffs 32**

Absorbent articles such as taped diapers, diaper pants or adult incontinence pants may typically further comprise cuff components 30 that improve the fit of the article around the legs of the wearer. Such cuffs typically comprise barrier leg cuffs 34 (also referred to as inner cuffs) and gasketing cuffs 32 (also referred to as outer cuffs). The cuffs 30 may comprise a piece of material 36, typically a nonwoven, which is one side partially bonded to the article and on the other side can be partially raised away from the topsheet and thus stand up from the plane defined by the topsheet as shown for example in Fig. 3. Both parts of the cuffs may be advantageously elasticized. The raised part of the cuff components is referred to herein as barrier leg cuffs 34 and can provide improved containment of liquids and other body exudates approximately at the junction of the torso and legs of the wearer. The barrier leg cuffs 34 extend at least partially between the front edge and the back edge of the absorbent article on opposite sides of the longitudinal axis and are at least present adjacent to the center point C of the article.

The barrier leg cuffs 34 may be delimited by a proximal edge 37 joined to the rest of the article, typically the topsheet, and a free terminal edge 38 intended to contact and form a seal with the wearer's skin. The barrier leg cuffs 34 may be joined at the proximal edge 37 with the chassis of the article by a bond which may be made for example by adhesive bonding, fusion bonding or combination of known bonding means, for example as disclosed in WO2014/168810A1 (Bianchi et al.). The bond at the proximal edge 37 may be continuous or intermittent.

The barrier leg cuffs 34 can be integral with (i.e. formed from) the topsheet or the backsheet, or more typically be formed from a separate material joined to the rest of the article. Typically the material of the barrier leg cuffs may extend through the whole length of the article but is "tack bonded" to the topsheet towards the front edge and back edge of the article so that in these sections the barrier leg cuff material remains flush with the topsheet. Each barrier leg cuff 34 may comprise one, two or more elastic strings 35 close to its free terminal edge 38 to provide a better seal.

The gasketing cuffs 32, which are formed in the same plane as the chassis of the absorbent article, may be at least partially enclosed between the topsheet and the backsheet, and are typically placed further laterally outwardly relative to the barrier leg cuffs 34. The gasketing cuffs 32 can provide a better seal around the thighs of the wearer. Usually each gasketing leg cuff 32 will comprise one or more elastic string or elastic element 33 comprised in the chassis of the diaper for example between the topsheet and backsheet in the area of the leg openings. Typically the barrier leg cuffs 34 are disposed more internally than the gasketing cuffs 32. The barrier leg cuffs are thus also referred to as inner cuffs and the gasketing cuffs as outer cuffs.

For example, US3,860,003 describes a disposable diaper which provides a contractible leg opening having a side flap and one or more elastic members to provide an elasticized leg cuff (a gasketing cuff). US4,808,178 (Aziz) and US4,909,803 (Aziz) describe disposable diapers having "stand-up" elasticized flaps (barrier leg cuffs) which improve the containment of the leg regions. US4,695,278 (Lawson) and US4,795,454 (Dragoo) describe disposable diapers having dual cuffs, including gasketing cuffs and barrier leg cuffs. All or a portion of the barrier leg and/or gasketing cuffs may be treated with a lotion.

**Other components**

The absorbent articles of the invention can further comprise any other typical components known for the intended purpose of the article which may or may not be illustrated in the Figures, such as a transverse barrier element extending across the topsheet to form a receptacle for bowel movement, a lotion application on the topsheet, a wetness indicator 70 comprising a pH indicator disposed between the absorbent core and the backsheet, etc. These components are well-known in the art and will not be further discussed herein. Reference is made to WO2014/093310 where several examples of these components are disclosed in more details.

The absorbent article may also comprise at least one elastic waist band (also called elastic waist feature) disposed parallel to and along the back edge of the article. The articles may also comprise such an elastic waist feature parallel to and along the front edge of the article. Such waistbands help providing improved fit and containment at the back and/or front edge of the article. The elastic waist feature is generally intended to elastically expand and contract to dynamically fit the wearer's waist. The elastic waist feature may be constructed in a number of different configurations. Non-limiting examples of back and front waistbands can be found in WO2012/177400 and WO2012/177401 (Lawson), and US4,515,595, US4,710,189, US5,221,274 and US6,336,922 (VanGompel et al.).

**Relations between the layers and components**

Typically, adjacent layers will be joined together using conventional bonding method such as adhesive coating via slot coating or spraying on the whole or part of the surface of the layer, or thermo-bonding, or pressure bonding or combinations thereof. Most of the bonding between components is for clarity and readability not represented in the Figure. Bonding between the layers of the article should be considered to be present unless specifically excluded. Adhesives may be typically used to improve the adhesion of the different layers, for example between the backsheet and the core wrap. The adhesives used may be any standard hotmelt glue as known in the art. The individual components may be converted into an absorbent article according to any of the processes known in the art.

**Packages**

A plurality of articles according to the invention may be packaged in a package for transport and sale. At least 50% of the articles in the package may be according to the invention, and preferably substantially all the articles in the package. The articles may be folded and packaged according to any conventional packages is known in the art. The package may be for example a plastic bag or a cardboard box. Diapers may typically bi-folded along the transversal axis and the ears folded inwardly before being packaged. The absorbent articles may be packed under compression so as to reduce the size of the packages, while still providing an adequate amount of absorbent articles per package. By packaging the absorbent articles under compression, caregivers can easily handle and store the packages, while also providing distribution and inventory savings to manufacturers owing to the size of the packages.

The absorbent articles may thus be packaged compressed at an In-Bag Compression Rate of at least 10%, in particular of from 10% to 50%, in particular from 20% to 40%. The "In-Bag Compression Rate" as used herein is one minus the height of a stack of 10 folded articles measured while under compression within a bag ("In-Bag Stack Height") divided by the height of a stack of 10 folded articles of the same type before compression, multiplied by 100; i.e. (1-In-Bag Stack Height/stack height before compression) * 100, reported as a percentage. Of course, the stack in the bag does not need to have exactly 10 articles, rather the value measured for the height of stack of article in the package is divided by the number of articles in the stack and then multiplied by 10. The method used to measure the In-Bag Stack Height is described in further details in the Test Procedures. The articles before compression may be typically sampled from the production line between the folding unit and the stack packing unit. The stack height before compression is measured by taking 10 articles before compression and packing, and measuring their stack height as indicated for the IBSH.

Packages of the absorbent articles of the present disclosure may in particular have an In-Bag Stack Height of less than 110 mm, less than 105 mm, less than 100 mm, less than 95 mm, less than 90 mm, specifically reciting all 0.1 mm increments within the specified ranges and all ranges formed therein or thereby, according to the In-Bag Stack Height Test described herein. For each of the values indicated in the previous sentence, it may be desirable to have an In-Bag Stack Height of greater than 60, or greater than 70 mm, or greater than 75 mm, or greater than 80 mm. Alternatively, packages of the absorbent articles of the present disclosure may have an In-Bag Stack Height of from 60 mm to 110 mm, from 75 mm to 110 mm, from 80 mm to 110 mm, from 80 mm to 105 mm, or from 80 mm to 100 mm, specifically reciting all 0.1 mm increments within the specified ranges and all ranges formed therein or thereby, according to the In-Back Stack Height Test described herein.

**Test procedures**

The values indicated herein are measured according to the methods indicated herein below, unless specified otherwise. All measurements are performed at 21°C ± 2°C and 50% ± 5% RH, unless specified otherwise. All samples should be kept at least 24 hours in these conditions to equilibrate before conducting the tests, unless indicated otherwise. All measurements should be reproduced on at least 4 samples and the average value obtained indicated, unless otherwise indicated.

Centrifuge Retention Capacity (CRC)

The CRC measures the liquid absorbed by the superabsorbent polymer particles for free swelling in excess liquid. The CRC is measured according to EDANA method WSP 241.2.R3 (12).

Dry Absorbent Core Caliper Test

This test may be used to measure the caliper of the absorbent core (before use i.e. without fluid loading) in a standardized manner.

Equipment: Mitutoyo manual caliper gauge with a resolution of 0.01 mm, or equivalent instrument.

Contact Foot: Flat circular foot with a diameter of 17.0 mm (± 0.2 mm). A circular weight may be applied to the foot (e.g., a weight with a slot to facilitate application around the instrument shaft) to achieve the target weight. The total weight of foot and added weight (including shaft) is selected to provide 2.07 kPa (0.30 psi) of pressure to the sample.

The caliper gauge is mounted with the lower surface of the contact foot in an horizontal plane so that the lower surface of the contact foot contacts the center of the flat horizontal upper surface of a base plate approximately 20 x 25 cm. The gauge is set to read zero with the contact foot resting on the base plate.

Ruler: Calibrated metal ruler graduated in mm.

Stopwatch: Accuracy 1 second.

Sample preparation: The core is conditioned at least 24 hours as indicated above.

Measurement procedure: The core is laid flat with the bottom side, i.e. the side intended to be placed towards the backsheet in the finished article facing down. The point of measurement (e.g. the center point C or P point P) is carefully drawn on the top side of the core taking care not to compress or deform the core.

The contact foot of the caliper gauge is raised and the core is placed flat on the base plate of the caliper gauge with the top side of the core up so that when lowered, the center of the foot is on the marked measuring point.

The foot is gently lowered onto the article and released (ensure calibration to "0" prior to the start of the measurement). The caliper value is read to the nearest 0.01 mm, 10 ± 1 seconds after the foot is released.

The procedure is repeated for each measuring point. If there is a fold at the measuring point, the measurement is done in the closest area to this point but without any folds. Ten articles are measured in this manner for a given product and the average caliper is calculated and reported with an accuracy of one tenth mm.

Absorbent Article Caliper Test

The Absorbent Article Caliper Test can be performed as for the Dry Absorbent Core Caliper Test with the difference that the caliper of the finished absorbent article is measured instead of the caliper of the core. The point of measurement may be the intersection C of the longitudinal axis 80 and transversal axis 90 of the absorbent article. If the absorbent articles were provided folded and/or in a package, the articles to be measured are unfolded and/or removed from the center area of the package. If the package contains more than 4 articles, the outer most two articles on each side of the package are not used in the testing. If the package contains more than 4 but fewer than 14 articles, then more than one package of articles is required to complete the testing. If the package contains 14 or more articles, then only one package of articles is required to perform the testing. If the package contains 4 or fewer articles then all articles in the package are measured and multiple packages are required to perform the measurement. Caliper readings should be taken 24 $\pm$ 1 hours after the article is removed from the package, unfolded and conditioned. Physical manipulation of product should be minimal and restricted only to necessary sample preparation.

Any elastic components of the article that prevent the article from being laid flat under the caliper foot are cut or removed. These may include leg cuffs or waistbands. Pant-type articles are opened or cut along the side seams as necessary. Apply sufficient tension to flatten out any folds/wrinkles. Care is taken to avoid touching and/or compressing the area of measurement.

Water Retention Value Measurement Procedure

The following procedure is utilized to determine the water retention value of fibers using a centrifugal method. A sample of 0.35 $\pm$ 0.05 grams of fibers is soaked in a covered container with 100 mL distilled water at 23 $\pm$ 2°C for 17 hours. The soaked fibers are collected on a filter and transferred to a US standard 80-mesh wire basket supported 40 mm above a 60-mesh screened bottom of a centrifuge tube. The tube is covered with a plastic cover and the sample is centrifuged at a relative centrifuge acceleration of 1600 $\pm$ 100 gravities (15.7 $\pm$ 1.0 km/s$^2$) for 20 minutes. The centrifuged fibers are then removed from the basket and weighed. The weighed fibers are dried to a constant weight at 105°C in a forced-air oven located in a controlled temperature and humidity environment at 23 $\pm$ 2°C and 50 $\pm$ 5% RH. The water retention value (WRV) is calculated as follows:

$$WRV = \frac{(W - D)}{D} \times 100$$

where

W = wet weight of centrifuged fibers
D = dry weight of centrifuged fibers, and
W - D = weight of absorbed water

In-Bag Stack Height Test

The In-Bag stack height of a package of absorbent articles is determined as follows:
Equipment: A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within $\pm$ 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 $\pm$ 1 gramforce (8.34 N) on the absorbent article package, which may be achieved by placing a suitable weight on the center of the non-package-contacting top surface of the horizontal sliding plate so that the total mass of the sliding plate plus added weight is 850 $\pm$ 1grams. Such a testing apparatus is for example illustrated on Fig. 19 of US2008/0312624A1.

Test Procedure: Absorbent article packages are equilibrated at 21 $\pm$ 2 °C and 50 $\pm$ 5% relative humidity prior to measurement. The horizontal sliding plate is raised and an absorbent article package is placed centrally under the horizontal sliding plate in such a way that the absorbent articles within the package are in a horizontal orientation. Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within $\pm$ 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) $\times$ 10 is calculated and reported to within $\pm$ 0.5 mm.

**Misc**

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of" which excludes any element, step or ingredient not mentioned which materially affect the way the feature performs its function, and the term "consisting of" which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

Unless indicated otherwise, the description and claims refer to the absorbent core and article before use (i.e. dry, and not loaded with a fluid) and conditioned at least 24 hours at 21 °C +/- 2 °C and 50 +/- 5% Relative Humidity (RH).

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

**Claims**

1. An absorbent article (20) for personal hygiene having a front edge (10), a back edge (12) and a longitudinal axis (80) extending from the front edge to the back edge, the absorbent article comprising:

    - a fluid permeable topsheet (24) on a wearer-facing side;
    - a fluid-impermeable backsheet (25) on a garment-facing side;
    - an absorbent core (28) comprising an absorbent material, between the topsheet and the backsheet;
    - an intermediate layer (54) between the topsheet and the absorbent core, the intermediate layer comprising a fibrous material, wherein the intermediate layer comprises:

        - a first and second longitudinally-extending channels (86a,b) substantially free of fibrous material; wherein the first channel (86a) is on one side of the longitudinal axis and the second channel (86b) is on the other side of the longitudinal axis;
        - a central area (82) comprising fibrous material between the first and second longitudinally-extending channels;
        - a back pocket (87) substantially free of fibrous material and disposed backward relative to the first and second channels;
        **characterized in that** the first and second channels are connected to the back pocket to form an unitary area (86-87) substantially free of fibrous material.

2. An absorbent article according to the preceding claim, wherein the unitary area formed by the first and second channels and the back pocket is entirely surrounded by fibrous material and wherein the intermediate layer comprises the following areas containing fibrous material: a front area (84) disposed forward of the first and second channels, a back area (85) disposed backward of the back pocket, and a first and second lateral outside areas (81, 83) disposed outwardly of the first and second channels respectively and the back pocket.

3. An absorbent article according to the preceding claim, wherein the minimum distance between the unitary area and any of the outer edges of the fibrous layer is of at least 5 mm.

4. An absorbent article according to any of the preceding claims, wherein the combined surface of the first and second channels and the back pocket represents from 5% to 50% of the overall surface of the intermediate layer,

5. An absorbent article according to any of the preceding claims, wherein the fibrous material has a Water Retention Value of from 2 to 60, as measured by the Water Retention Value Measurement Procedure as indicated herein.

6. An absorbent article according to any of the preceding claims, wherein the fibrous material comprises or consists of cellulosic fibers.

7. An absorbent article according to the preceding claim, wherein the fibrous material comprises or consists of cross-linked cellulose fibers.

8. An absorbent article according to any of the preceding claims wherein the first and second channels of the intermediate layer are at least partially curved.

9. An absorbent article according to any of the preceding claims, wherein the central area of the intermediate layer has a basis weight of from 50 to 300 g/m$^2$.

10. An absorbent article according to the preceding claim, wherein the length (d2+d3) of the unitary area formed by the channels and the back pockets ranges from 10% to 70% of the length of the absorbent article (L).

11. An absorbent article according to any of the preceding claims, wherein the absorbent core comprises a core wrap comprising a top side and a bottom side enclosing the absorbent material, and wherein the absorbent material comprises superabsorbent particles optionally mixed with cellulose fibers.

12. An absorbent article according to the preceding claim, wherein the absorbent material does not comprise cellulose fibers, in particular wherein the absorbent material consists of superabsorbent particles and optionally an adhesive that at least partially immobilize the superabsorbent particles within the core wrap.

13. An absorbent article according to any of the preceding claims, wherein the absorbent core comprises at least two channel areas (26a, 26b) and these channel areas are at least partially superposed with the channels of the intermediate layer.

14. An absorbent article according to the preceding claim, wherein the absorbent core comprises a core wrap having a top side (16) and a bottom side (16') enclosing the absorbent material, and the channel areas of the absorbent core are substantially free of absorbent material, and the top side of the core wrap (16) is continuously or intermittently attached to the bottom side (16') of the core wrap through the channel areas.

15. A package comprising a plurality of absorbent articles according to any of the preceding claims.

**Patentansprüche**

1. Absorptionsartikel (20) für Körperhygiene mit einem vorderseitigen Rand (10), einem hinterseitigen Rand (12) und einer Längsachse (80), die sich von dem vorderseitigen Rand zum hinterseitigen Rand erstreckt, wobei der Absorptionsartikel Folgendes umfasst:

   - eine fluiddurchlässige Oberschicht (24) auf einer trägerseitigen Seite;
   - eine fluidundurchlässige Unterschicht (25) auf einer zum Kleidungsstück zeigenden Seite;
   - einen Absorptionskern (28), der zwischen der Oberschicht und der Unterschicht angeordnet ist;
   - eine Zwischenschicht (54) zwischen der Oberschicht und dem Absorptionskern, wobei die Zwischenschicht ein Fasermaterial umfasst, wobei die Zwischenschicht Folgendes umfasst:

      - einen ersten und einen zweiten, sich in Längsrichtung erstreckenden Kanal (86a, b), der im Wesentlichen frei von Fasermaterial ist; wobei sich der erste Kanal (86a) auf einer Seite der Längsachse und der zweite Kanal (86b) auf der anderen Seite der Längsachse befindet;
   - einen Zentralbereich (82), der Fasermaterial zwischen dem ersten und dem zweiten, sich in Längsrichtung erstreckenden Kanal umfasst;
   - eine rückseitige Tasche (87), die im Wesentlichen frei von Fasermaterial ist und in Bezug zu dem ersten und dem zweiten Kanal rückwärts angeordnet ist;
   **dadurch gekennzeichnet, dass** der erste und der zweite Kanal mit der rückseitigen Tasche verbunden sind, um einen einstückigen Bereich (86-87) zu bilden, der im Wesentlichen frei von Fasermaterial ist.

2. Absorptionsartikel nach dem vorstehenden Anspruch, wobei der einstückige Bereich, der durch den ersten und zweiten Kanal und die rückseitige Tasche gebildet wird, vollständig von Fasermaterial umgeben ist, und wobei die Zwischenschicht die folgenden Fasermaterial enthaltenden Bereiche umfasst: einen vorderseitigen Bereich (84), der vor dem ersten und zweiten Kanal angeordnet ist, einen rückseitigen Bereich (85), der hinter der rückseitigen Tasche angeordnet ist, und einen ersten und zweiten seitlichen Außenbereich (81, 83), der außerhalb des ersten bzw. zweiten Kanals und der rückseitigen Tasche angeordnet ist.

3. Absorptionsartikel nach dem vorstehendem Anspruch, wobei der Mindestabstand zwischen dem einstückigen Bereich und einem der Außenränder der Faserschicht mindestens 5 mm beträgt.

4. Absorptionsartikel nach einem der vorstehenden Ansprüche, wobei die kombinierte Oberfläche des ersten und des zweiten Kanals und der rückseitigen Tasche von 5 % bis 50 % der Gesamtoberfläche der Zwischenschicht darstellt,

5. Absorptionsartikel nach einem der vorstehenden Ansprüche, wobei das Fasermaterial ein Wasserrückhaltevermögen von 2 bis 60 aufweist, gemessen durch das Wasserrückhaltevermögen-Messverfahren wie hierin angegeben.

6. Absorptionsartikel nach einem der vorstehenden Ansprüche, wobei das Fasermaterial Zellulosefasern umfasst oder aus diesen besteht.

7. Absorptionsartikel nach einem der vorstehenden Ansprüche, wobei das Fasermaterial quervernetzte Zellulosefasern umfasst oder aus diesen besteht.

8. Absorptionsartikel nach einem der vorstehenden Ansprüche, wobei der erste und der zweite Kanal der Zwischenschicht zumindest teilweise gekrümmt sind.

9. Absorptionsartikel nach einem der vorstehenden Ansprüche, wobei der Mittelbereich der Zwischenschicht ein Grundgewicht von 50 bis 300 g/m$^2$ aufweist.

10. Absorptionsartikel nach dem vorstehenden Anspruch, wobei die Länge (d2+d3) des einstückigen Bereichs, der durch die Kanäle und die rückseitigen Taschen gebildet wird, im Bereich von 10 % bis 70 % der Länge des Absorptionsartikel (L) liegt.

11. Absorptionsartikel nach einem der vorstehenden Ansprüche, wobei der Absorptionskern eine Kernumwicklung umfasst, die eine Oberseite und eine Unterseite umfasst, die das Absorptionsmaterial umschließen, und wobei das Absorptionsmaterial superabsorbierende Teilchen umfasst, die wahlweise mit Zellulosefasern gemischt sind.

12. Absorptionsartikel nach dem vorstehenden Anspruch, wobei das Absorptionsmaterial keine Zellulosefasern umfasst, wobei insbesondere das Absorptionsmaterial aus superabsorbierenden Teilchen und wahlweise einem Klebstoff besteht, der die superabsorbierenden Teilchen innerhalb der Kernumwicklung zumindest teilweise immobilisieren.

13. Absorptionsartikel nach einem der vorstehenden Ansprüche, wobei der Absorptionskern mindestens zwei Kanalbereiche (26a, 26b) umfasst und diese Kanalbereiche zumindest teilweise mit den Kanälen der Zwischenschicht übereinander angeordnet sind.

14. Absorptionsartikel nach dem vorstehenden Anspruch, wobei der Absorptionskern eine Kernumwicklung mit einer Oberseite (16) und einer Unterseite (16') umfasst, die das Absorptionsmaterial umschließen, und die Kanalbereiche des Absorptionskerns im Wesentlichen frei von Absorptionsmaterial sind und die Oberseite der Kernumwicklung (16) an der Unterseite (16') durch die Kanalbereiche ununterbrochen oder unterbrochen angebracht ist.

15. Verpackung, umfassend eine Vielzahl von Absorptionsartikeln nach einem der vorstehenden Ansprüche.

## Revendications

1. Article absorbant (20) pour hygiène personnelle ayant un bord avant (10), un bord arrière (12) et un axe longitudinal (80) s'étendant du bord avant au bord arrière, l'article absorbant comprenant :

   - une feuille de dessus perméable aux fluides (24) sur un côté faisant face au porteur ;
   - une feuille de fond imperméable aux fluides (25) sur un côté faisant face au vêtement ;
   - une âme absorbante (28) comprenant un matériau absorbant, entre la feuille de dessus et la feuille de fond ;
   - une couche intermédiaire (54) entre la feuille de dessus et l'âme absorbante, la couche intermédiaire comprenant un matériau fibreux, dans lequel la couche intermédiaire comprend :

     - des premier et deuxième canaux s'étendant longitudinalement (86a, b) essentiellement dépourvus de matériau fibreux ; dans lequel le premier canal (86a) est sur un côté de l'axe longitudinal et le deuxième canal (86b) est sur l'autre côté de l'axe longitudinal ;
     - une zone centrale (82) comprenant un matériau fibreux entre les premier et deuxième canaux s'étendant longitudinalement ;
     - une poche arrière (87) essentiellement dépourvue de matériau fibreux et disposée vers l'arrière par rapport aux premier et deuxième canaux ;
     **caractérisé en ce que** les premier et deuxième canaux sont reliés à la poche arrière pour former une zone d'un seul tenant (86-87) essentiellement dépourvue de matériau fibreux.

2. Article absorbant selon la revendication précédente, dans lequel la zone d'un seul tenant formée par les premier et deuxième canaux et la poche arrière est entièrement entourée par un matériau fibreux et dans lequel la couche intermédiaire comprend les zones suivantes contenant un matériau fibreux : une zone avant (84) disposée vers l'avant des premier et deuxième canaux, une zone arrière (85) disposée vers l'arrière de la poche arrière, et des première et deuxième zones extérieures latérales (81, 83) disposées vers l'extérieur des premier et deuxième canaux respectivement et de la poche arrière.

3. Article absorbant selon la revendication précédente, dans lequel la distance minimale entre la zone d'un seul tenant et n'importe lequel des bords externes de la couche fibreuse est d'au moins 5 mm.

4. Article absorbant selon l'une quelconque des revendications précédentes, dans lequel la surface combinée des premier et deuxième canaux et de la poche arrière représente de 5 % à 50 % de la surface globale de la couche intermédiaire,

5. Article absorbant selon l'une quelconque des revendications précédentes, dans lequel le matériau fibreux a un taux de rétention de l'humidité allant de 2 à 60, tel que mesuré par la procédure de mesure de taux de rétention de l'humidité telle qu'indiquée ici.

6. Article absorbant selon l'une quelconque des revendications précédentes, dans lequel le matériau fibreux comprend ou est constitué de fibres cellulosiques.

7. Article absorbant selon la revendication précédente, dans lequel le matériau fibreux comprend ou est constitué de fibres de cellulose réticulée.

8. Article absorbant selon l'une quelconque des revendications précédentes, dans lequel les premier et deuxième canaux de la couche intermédiaire sont au moins partiellement incurvés.

9. Article absorbant selon l'une quelconque des revendications précédentes, dans lequel la zone centrale de la couche intermédiaire a une masse surfacique allant de 50 à 300 g/m².

10. Article absorbant selon la revendication précédente, dans lequel la longueur (d2+d3) de la zone d'un seul tenant formée par les canaux et les poches arrière va de 10 % à 70 % de la longueur de l'article absorbant (L).

11. Article absorbant selon l'une quelconque des revendications précédentes, dans lequel l'âme absorbante comprend une enveloppe d'âme comprenant un côté supérieur et un côté inférieur enfermant le matériau absorbant, et dans lequel le matériau absorbant comprend des particules de superabsorbant éventuellement mélangées à des fibres de cellulose.

12. Article absorbant selon la revendication précédente, dans lequel le matériau absorbant ne comprend pas de fibres de cellulose, en particulier dans lequel le matériau absorbant est constitué de particules de superabsorbant et éventuellement d'un adhésif qui immobilisent au moins partiellement les particules de superabsorbant au sein de l'enveloppe d'âme.

13. Article absorbant selon l'une quelconque des revendications précédentes, dans lequel l'âme absorbante comprend au moins deux zones de canal (26a, 26b) et ces zones de canal sont au moins partiellement superposées aux canaux de la couche intermédiaire.

14. Article absorbant selon la revendication précédente, dans lequel l'âme absorbante comprend une enveloppe d'âme ayant un côté supérieur (16) et un côté inférieur (16') enfermant le matériau absorbant, et les zones de canal de l'âme absorbante sont essentiellement dépourvues de matériau absorbant, et le côté supérieur de l'enveloppe d'âme (16) est fixé de façon continue ou intermittente au côté inférieur (16') de l'enveloppe d'âme par l'intermédiaire des zones de canal.

15. Conditionnement comprenant une pluralité d'articles absorbants selon l'une quelconque des revendications précédentes.

# Fig. 1

**Fig. 2**

Fig. 3

# Fig. 4

EP 3 315 106 B1

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9423761 A, Payne
- US 5486166 A
- US 5490846 A, Bishop
- WO 201493323 A, Bianchi
- US 20080312621 A
- US 20080312622 A, Hundorf
- WO 9917679 A, Everett
- WO 201531225 A
- WO 201531229 A
- WO 201531243 A
- WO 201531256 A, Roe
- WO 2015031225 A, Roe
- WO 2015031243 A, Roe
- US 2008312622 A1, Hundorf
- US 5549791 A
- US 5137537 A
- WO 9534329 A
- US 2007118087 A
- US 6632504 B, Gillespie
- WO 2011163582 A
- US 3929135 A
- US 4324246 A
- US 4342314 A
- US 4463045 A
- US 5006394 A
- US 4609518 A
- US 4629643 A
- US 20140121623 A1
- US 20140121621 A1
- US 20140121624 A1
- US 20140121625 A1
- US 6645569 B
- US 6863933 B
- US 2003148684 A
- US 2005008839 A, Cramer
- US 7112621 B, Rohrbaugh
- US 5607760 A
- US 5609587 A
- US 5643588 A
- US 5968025 A
- US 6716441 B
- WO 9524173 A
- US 7786341 B
- EP 149880 A, Kwok
- US 20030105190 A, Diehl
- WO 200071067 A, KIM DOO-HONG
- WO 2008155699 A, Hundorf
- US 2006024433 A, Blessing
- US 20080312617 A
- US 20100051166 A1, Hundorf
- US 20120312491 A, Jackels
- WO 2014093129 A1, Roe
- US 7744576 B
- US 20110268932 A1
- US 20110319848 A1
- US 20110250413 A1
- WO 07047598 A
- WO 07046052 A
- WO 2009155265 A
- WO 2009155264 A
- WO 2006083584 A
- US 2014005622 A1
- WO 2015041784 A, Peri
- US 3848594 A
- US 4662875 A
- US 4846815 A
- US 4894060 A
- US 4946527 A
- US 5151092 A
- US 5221274 A, Buell
- US 6432098 B
- US 4963140 A, Robertson
- US 4699622 A
- US 5242436 A
- US 5499978 A
- US 550786 A
- US 5591152 A
- WO 2014022362 A1
- WO 2014022652 A1
- US 5837352 A
- US 5843056 A
- WO 2014168810 A1, Bianchi
- US 3860003 A
- US 4808178 A, Aziz
- US 4909803 A, Aziz
- US 4695278 A, Lawson
- US 4795454 A, Dragoo
- WO 2014093310 A
- WO 2012177400 A
- WO 2012177401 A, Lawson
- US 4515595 A
- US 4710189 A
- US 6336922 B, VanGompel
- US 20080312624 A1